Europäisches Patentamt

European Patent Office

Office européen des brevets

(19)

(11) Veröffentlichungsnummer: **0 049 410**
**B1**

(12) **EUROPÄISCHE PATENTSCHRIFT**

(45) Veröffentlichungstag der Patentschrift:
01.04.87

(51) Int. Cl.⁴: **C 12 N 1/00**, C 12 N 1/14,
C 12 N 1/20

(21) Anmeldenummer: 81107449.1

(22) Anmeldetag: 19.09.81

(54) **Wässriges Nährmedium zur Anzucht von Mikroorganismen.**

(30) Priorität: 07.10.80 DE 3038229

(43) Veröffentlichungstag der Anmeldung:
14.04.82 Patentblatt 82/15

(45) Bekanntmachung des Hinweises auf die Patenterteilung:
01.04.87 Patentblatt 87/14

(84) Benannte Vertragsstaaten:
AT BE CH DE FR GB IT LI LU NL SE

(56) Entgegenhaltungen:
GB - A - 444 929
GB - A - 558 889
GB - A - 695 378
US - A - 1 425 065
US - A - 2 474 339

CHEMICAL ABSTRACTS, Band 92, Nr. 1, 7. Januar 1980,
Seite 460, Nr. 4727q, Columbus, Ohio, USA
CHEMICAL ABSTRACTS, Band 93, Nr. 1, 7. Juli 1980,
Seite 601, Nr. 6402z, Columbus, Ohio, USA
CHEMICAL ABSTRACTS; Band 87, Nr. 15, 10. Oktober
1977, Seite 486, Nr. 116379f, Columbus, Ohio, USA
CHEMICAL ABSTRACTS, Band 55, Nr. 2, 23. Januar
1961, Spalte 2007c,d, Columbus, Ohio, USA
CHEMICAL ABSTRACTS, Band 52, Nr. 9, 10. März 1958,
Spalte 7433c, Columbus, Ohio, USA

(73) Patentinhaber: SCHERING AKTIENGESELLSCHAFT
Berlin und Bergkamen,
Müllerstrasse 170/178 Postfach 65 03 11,
D-1000 Berlin 65 (DE)

(72) Erfinder: Weber, Alfred, Dr., Schützallee 56,
D-1000 Berlin 37 (DE)
Erfinder: Kennecke, Mario, Dr., Taubertstrasse 31 F,
D-1000 Berlin 33 (DE)
Erfinder: Rippe, Manfred, Dr.,
Friedrich-Goerdeler-Strasse 12, D-4619 Bergkamen (DE)
Erfinder: Cejka, Alena, Dr., Bonifatiusstrasse 4,
D-4600 Dortmund (DE)

(56) Entgegenhaltungen: (Fortsetzung)
ORGANIC SYNTHESES, COLLECTIVE VOLUME I, 1946,
A.H. Blatt, New York, USA R.A. GORTNER et al.:
"L-Cystine", Seiten 194-196

## Beschreibung

Die Erfindung betrifft das in dem Patentanspruch 1 gekennzeichnete wässrige Nährmedium zur Anzucht von Mikroorganismen und insbesondere die in den Patentansprüchen 2 und 3 gekennzeichneten bevorzugten Ausführungsformen desselben.

Zur Anzucht nicht autotropher Mikroorganismen in der Fermentationsindustrie verwendet man Nährmedien, die die erforderlichen Kohlenstoffquellen – in der Regel Kohlehydrate – enthalten. Einige Mikroorganismen – insbesondere Hefen – sind in der Lage, sich in derartigen Nährmedien zu vermehren, die weitgehend frei von anderen Medienbestandteilen als Kohlehydraten sind. Es ist bekannt, dass man das Wachstum und gegebenenfalls die Gärfähigkeit derartiger Mikroorganismen erhöhen kann, wenn man den Nährmedien zusätzlich noch Eiweisshydrolysate als Stickstoffquellen zusetzt (C.A. 52, 1958, 7433c, C.A. 55, 1961, 2007c, C.A. 87, 1977, 116379f, C.A. 92, 1980, 4727q, C.A. 93, 1980, 6402z, GB-A-444 929, GB-A-558 889, GB-A-695 378, US-A-1 425 065 und US-A-2 474 339.)

Die meisten Mikroorganismen benötigen aber zur Anzucht Nährmedien, die neben den erforderlichen Kohlenstoffquellen und Stickstoffquellen zusätzlich noch komplexe Nährmedienzusätze, wie Cornsteep-Liquor oder Hefeextrakt, die die erforderlichen Wuchsstoffe und Vitamine enthalten. (Hans-Jürgen Rehm: Einführung in die industrielle Mikrobiologie, Springer Verlag, Berlin etc. 1971, Seite 21; Gerhard Drews: Mikrobiologisches Praktikum für Naturwissenschaftler, Springer Verlag, Berlin etc. Seite 1 bis 6).

Es wurde nun gefunden, dass man diese Wuchsstoffe und Vitamine enthaltenden komplexen Nährmedienzusätze ganz oder zumindest teilweise durch die in den Patentansprüchen gekennzeichneten kohlehydratfreien und 0,2 bis 2,0% Cystin enthaltendes Proteinhydrolysat-Lösungen ersetzen kann. Dies ist für den Fachmann überraschend, da dieser annehmen musste, dass eine aus Hufen, Federn oder Haaren dargestellte Proteinhydrolysat-Lösung lediglich als Substitut für organische Stickstoffquellen für die Mikroorganismen verwendet werden könne, dass es aber ungeeignet wäre um die Wuchsstoffe und Vitamine enthaltenden Nährmedienzusätze zu ersetzen.

Im Vergleich zu den bekannten wässrigen Nährmedien haben die erfindungsgemässen Medien – abgesehen von der Tatsache, dass sie preisgünstiger sind, da zu ihrer Herstellung Hufe, Federn oder Haare verwendet werden können, die bislang nicht ausreichend gewerblich verwertet werden – oft noch den Vorteil, dass mit ihrer Hilfe eine höhere Zelldichte, eine höhere Produktausbeute, eine Verkürzung der Fermentationszeit oder eine Erhöhung der Substratkonzentration erzielt werden kann, auch, dass es mit Hilfe des erfindungsgemässen Verfahrens möglich ist, derartige proteinhaltigen Naturprodukte, in Biomasse wie zum Beispiel Hefe umzuwandeln, welche als eiweissreiche hochwertige Futtermittel sehr geeignet sind.

Die Herstellung der Proteinhydrolysat-Lösungen aus Hufen, Federn oder Haaren erfolgt vorzugsweise unter den gleichen Bedingungen, wie sie üblicherweise zur Hydrolyse von Proteinen mit Mineralsäuren angewendet werden. Die so hergestellten Proteinhydrolysate werden neutralisiert und anschliessend filtriert und gewünschtenfalls gegebenenfalls eingeengt. Die so erhaltenen Proteinhydrolysat-Lösungen haben gegenüber anderen komplexen Medienzusätzen – wie zum Beispiel Cornsteep-Liquor – den nicht unbeträchtlichen Vorzug, dass sie klare Lösungen sind und demzufolge genau dosiert werden und sterilfiltriert werden können.

Die in den nachfolgenden Ausführungsbeispielen verwendete Proteinhydrolysat-Lösung wurde aus Federnmehl gemäss der Vorschrift von Organic Synthesis, Col. Vol. I, Seite 194 hergestellt, jedoch wurde nach erfolgter Hydrolyse mittels Ammoniak neutralisiert und sterilfiltriert. Das erhaltene Filtrat wurde ohne weitere Reinigung als «Proteinhydrolysat-Lösung» verwendet. Es ist aber auch möglich, die nach der Vorschrift von Organic Synthesis Col. Vol. I, Seite 194 hergestellte Proteinhydrolysat-Lösung selbst zu verwenden.

Beispiel 1

Ein Medium bestehend aus
0,5 % Cornsteep-Liquor
0,05% Glucose
0,3 % «Proteinhydrolysat-Lösung»
kann mit gleichem Ergebnis, wie das konventionellerweise verwendete Medium bestehend aus
0,5 % Cornsteep-Liquor
0,05% Glucose
0,1 % Hefeextrakt
zur Anzucht von Bacillus lentus ATCC 13 805 oder Arthrobacter simplex ATCC 6946 verwendet werden. Die so erhaltenen Kulturen werden beispielsweise zur delta$^1$-Dehydrierung von Steroiden verwendet.

Beispiel 2

Ein Medium bestehend aus
0,5% Cornsteep-Liquor
0,2% Glucose
1,0% «Proteinhydrolysat-Lösung»
ist zur Anzucht von Flavobacterium dehydrogenans ATCC 13 930 ebenso geeignet, wie das konventionellerweise verwendete Medium bestehend aus
0,5% Cornsteep-Liquor
0,2% Glucose
0,5% Hefeextrakt
Die so erhaltenen Kulturen werden beispielsweise zur Umwandlung von 3β-Acetoxy-delta$^5$-steroiden zu 3-Keto-delta$^4$-steroiden verwendet.

Beispiel 3

Ein Medium bestehend aus
0,25% «Proteinhydrolysat-Lösung»
1,25% Sojapuder

0,75% Kaliumdihydrogenphosphat
ist zur Anzucht von Curvularia lunata NRRL 2380 und Aspergillus occhraceus ATCC 1008 ebenso geeignet, wie das konventionellerweise verwendete Medium bestehend aus

1,0 % Cornsteep-Liquor
1,25% Sojapuder
0,75% Kaliumdihydrogenphosphat

Die so erhaltenen Kulturen werden beispielsweise zur 11β- oder 11alpha-Hydroxylierung von Steroiden verwendet.

## Patentansprüche

1. Wässriges Nährmedium zur Anzucht von Mikroorganismen, gekennzeichnet durch einen Gehalt an einer 0,2 bis 5,0% Cystin enthaltenden kohlehydratfreien Proteinhydrolysat-Lösung, welche durch Hydrolyse von Hufen, Federn oder Haaren mittels Mineralsäure, Neutralisation des Hydrolysats und Filtration desselben dargestellt wird, als Medienbestandteil.

2. Wässriges Nährmedium zur Anzucht von Mikroorganismen, gemäss Anspruch 1, dadurch gekennzeichnet, dass die Proteinhydrolysat-Lösung 10 bis 40 Gewichtsprozent Aminosäuren enthält.

3. Wässriges Nährmedium zur Anzucht von Mikroorganismen gemäss Anspruch 1 und 2, dadurch gekennzeichnet, dass es 0,1 bis 2,0% Proteinhydrolysat als Medienbestandteil enthält.

## Claims

1. Aqueous nutrient medium for the cultivation of microorganisms, characterised in that a component of the medium is a carbohydrate-free protein hydrolysate solution containing 0.2 to 5.0% cystein, which is prepared by hydrolysing horn, feathers or hair with mineral acid, and neutralising and filtering the hydrolysate.

2. Aqueous nutrient medium for the cultivation of microorganisms according to claim 1, characterised in that the protein hydrolysate solution contains 10 to 40% by weight of amino acids.

3. Aqueous nutrient medium for the cultivation of microorganisms according to claim 1 or 2, characterised in that it contains 0.1–2.0% protein hydrolysate as medium component.

## Revendications

1. Milieu nutritif aqueux pour la culture de micro-organismes, milieu caractérisé en ce qu'il contient, comme constituant, une solution d'hydrolysat de protéines dépourvue de glucides et renfermant de 0,2 à 5,0% de cystine, qui a été préparée par hydrolyse de sabots, de plumes ou de poils au moyen d'un acide minéral, neutralisation de l'hydrolysat et filtration de celui-ci.

2. Milieu nutritif aqueux pour la culture de micro-organismes, selon la revendication 1, milieu caractérisé en ce que la solution d'hydrolysat de protéines contient de 10 à 40% en poids d'aminoacides.

3. Milieu nutritif aqueux pour la culture de micro-organismes, selon l'une des revendications 1 et 2, qui contient de 0,1 à 2,0% de l'hydrolysat de protéines.